# FASCICULE DE BREVET EUROPEEN

(11) **EP 2 088 933 B1**
(45) Date de publication et mention de la délivrance du brevet: **21.04.2010**
(21) Numéro de dépôt: 07822325.2
(22) Date de dépôt: 07.11.2007
(51) Int. Cl.: A61B 17/00

(54) **DISPOSITIF DE DELIVRANCE DE BOUCHON D'OCCLUSION**
VORRICHTUNG ZUR ABGABE EINES VERSCHLUSSPFROPFES
DEVICE FOR DELIVERING AN OCCLUSION PLUG

(30) Priorité: 14.11.2006 FR 0654900
(43) Date de publication de la demande: 19.08.2009
(73) Titulaire: Protomed, 13916 Marseille (FR); Costecalde, Michel, 82000 Montauban (FR); Cau, Jérôme, 86000 Poitiers (FR)
(72) Inventeur: COSTECALDE, Michel, 82000 Montauban (FR); CAU, Jérôme, 86000 Poitiers (FR); MOURET, Frédéric, 13005 Marseille (FR); RAMOS CLAMOTTE, Joachim, 13001 Marseille (FR)
(74) Mandataire: Decobert, Jean-Pascal
(86) Numéro de dépôt international: PCT/EP2007/062015
(87) Numéro de publication internationale: WO 2008/058880

(56) Documents cités:
- WO-A-96/22123
- US-A1- 4 790 819
- US-A1- 5 326 350
- US-A1- 6 086 607
- US-A1- 2005 090 860

## Description

La présente invention concerne un dispositif de délivrance d'un bouchon dans un conduit du corps humain ou animal.

Elle s'applique particulièrement à la chirurgie vasculaire pour l'occlusion d'artères telles que les lombaires, les intercostales ou autres.

Par exemple, la chirurgie reconstructrice de l'aorte, après notamment un anévrisme aortique abdominal est une opération courante et particulièrement répandue chez les personnes de plus de 65 ans cela représente 150 000 à 200 000 opérations par an dans le monde.

La technique consiste à clamper le sac anévrismal, à l'ouvrir et à évacuer le caillot, puis enfin à remplacer l'anévrisme par une prothèse vasculaire. Les artères lombaires sont au nombre de cinq paires. Elles trouvent leur origine à la face postérieure de l'aorte abdominale. Les artères lombaires cheminent le long des vertèbres lombaires, en se divisant en deux branches : une branche dorso-spinale (postérieure) et une branche abdominale ou intercostale lombaire (antérieure).

Lors d'une opération d'un anévrisme de l'aorte, lorsque le chirurgien effectue la mise à plat de l'anévrisme, les artères lombaires sont à ce moment-là mises à jour. Elles sont alors une source de reflux sanguin : la présence d'une ouverture à l'air libre favorise le reflux puisqu'elle constitue une ouverture sur un environnement de basse pression, contrairement au réseau artériel qui est un environnement de haute pression (pression relative de l'ordre de 100 mmhg). Le sang reflue et provient d'un réseau artériel périphérique de l'aorte, celle-ci étant clampée.

La technique conventionnelle consiste à ligaturer les artères lombaires avec un fil de suture. Pendant le temps de ligature, les artères qui fuient peuvent être bouchées par les doigts des assistants du chirurgiens ou par des compresses. Normalement, seules quatre à six artères fuient. Les artères restent ligaturées après l'opération car il existe un réseau périphérique qui se substitue à la circulation de ces artères. Cette perte sanguine fait partie des plus importantes dans cette chirurgie. De plus, le temps pendant lequel est clampée l'aorte est critique pour le patient. Tout moyen de limiter ce temps ainsi que les pertes sanguines permet d'améliorer les suites opératoires pour le patient et ainsi d'opérer des patients plus tarés.

La problématique rencontrée en chirurgie conventionnelle est d'autant plus critique en chirurgie laparoscopique. Car il est impossible de boucher les artères par les doigts d'un assistant. La ligature est possible mais elle est délicate, puisqu'elle oblige à manipuler des aiguilles à l'aide d'instruments, à l'intérieur du ventre et requiert un temps précieux qui augmente considérablement les pertes sanguines (pertes pouvant atteindre un litre de sang) et le risque pour le patient.

Plusieurs solutions ont été proposées afin de limiter le temps et la difficulté nécessaire à l'occlusion de ces artères lombaires.

Le plus souvent, un bouchon d'occlusion est mis en place. Le bouchon est à l'état comprimé avant d'être introduit dans le vaisseau puis reprend sa forme originale expansée une fois mis en place.

II ne serait pas possible d'insérer un bouchon non comprimé en déformant les vaisseaux. En effet, les artères lombaires ont un faible diamètre compris entre 1 et 2,5 mm et présentent des parois fines et fragiles. La dilatation de l'artère au-delà de son diamètre normal afin d'y insérer un bouchon pourrait créer une rupture de l'artère et provoquer une hémorragie. De plus, ces artères peuvent présenter des calcifications qui les rendent extrêmement rigides. Il est donc difficile de les dilater.

*On connaît le document* WO 0019912 *qui décrit un dispositif pour l'occlusion d'une perforation transpariétale comprenant un bouchon comprimé dans un introducteur composé d'un tube externe de constriction de section constante et un tube interne pousseur. Le bouchon est à l'état comprimé dans l'introducteur. Si le bouchon reste comprimé trop longtemps avant sa mise en place, il y a un risque qu'il y ait une rémanence de forme et qu'il ne s'expanse pas correctement.*

*D'autres documents présentent des systèmes sensiblement identiques pour des utilisations plus complexes. Par exemple,* le document US 4,790,819 décrit un dispositif pour mettre en place un bouchon d'occlusion, sur lequel est basée la forme en deux parties de la revendication 1, ainsi que *le document* WO 94/26175 *qui décrit un dispositif pour mettre en place un bouchon d'occlusion dans un vaisseau sanguin dont l'extrémité est incurvée.*

*Les différents dispositifs connus présentent un seul tube constricteur recevant le bouchon déjà comprimé. L'inconvénient de ces dispositifs est le risque que le bouchon ne s'expanse pas correctement une fois mis en place du fait d'une rémanence de forme.*

Il existe donc le besoin de proposer un dispositif permettant la délivrance d'un bouchon d'occlusion qui garde ses propriétés élastiques de façon certaine.

A cet effet, l'invention propose un dispositif de délivrance d'un bouchon dans un conduit du corps humain ou animal comprenant un introducteur muni d'un canal de guidage et des moyens pour pousser le bouchon dans le canal caractérisé par le fait que ledit canal est configuré pour comprimer radialement le bouchon au cours de son déplacement vers l'extrémité distale du canal et que les moyens pour pousser le bouchon comprennent au moins deux tiges imbriquées aptes à coulisser l'une dans l'autre.

La compression est ainsi réalisée seulement quelques secondes avant sa mise en place en respectant son intégrité. Cela permet au bouchon de conserver toutes ses caractéristiques d'élasticité.

Avantageusement, pour comprimer le bouchon radialement au cours de son déplacement vers l'extrémité distale du canal, celui-ci est composé d'une succession de compartiments de section décroissante ou selon un autre mode de réalisation le canal a un profil en forme de tronc de cône.

Ainsi le fonctionnement des moyens pour pousser le bouchon conduira à la compression du bouchon dans le canal.

Avantageusement, le canal se situe à l'extrémité distale dudit introducteur dans un embout amovible. Ce mode de réalisation permet au dispositif d'être stérilisable ou d'être à usage unique.

Selon l'une des variantes préférées de l'invention, le dispositif est tel que :
ledit canal est composé d'une succession de compartiments de section décroissante.
ledit canal a un profil en forme de tronc de cône destiné à réduire la section dudit canal vers son extrémité distale.
le canal se situe à l'extrémité distale dudit introducteur au moins pour partie dans un embout amovible.
les moyens pour pousser le bouchon comportent des moyens de compression situés dans un appareil sur lequel l'embout est connectable et des moyens de poussée terminale situés dans l'introducteur.
la connexion entre l'introducteur et son embout amovible est réalisée par une baïonnette.
la connexion entre l'introducteur et son embout amovible est réalisée par vissage.
une tige possède une réduction de section à son extrémité distale
l'introducteur est muni d'un ergot destiné à faciliter le positionnement du dispositif en regard du conduit à occlure.
l'introducteur est muni à son extrémité distale d'une surface d'appui agrandie destinée à permettre l'appui dudit dispositif sur le pourtour du conduit à occlure.
les moyens pour pousser le bouchon comprennent un fluide intermédiaire destiné à transmettre l'effort des tiges imbriquées au bouchon.
l'extrémité distale dudit introducteur est munie d'un moyen d'évacuation destiné à éviter les surpressions.
ledit introducteur comprend un logement de stockage destiné à stocker les bouchons avant leur utilisation.
le logement de stockage comprend un barillet destiné à stocker et à distribuer les bouchons.

L'invention concerne aussi un procédé de mise en compression d'un bouchon caractérisé par le fait qu'on utilise un dispositif de délivrance tel que décrit précédemment et que le bouchon est introduit à l'extrémité proximale d'un embout amovible dans un canal, l'embout amovible est connecté à un appareil comportant des moyens de compression, une pression longitudinale est exercée sur l'embout amovible de sorte à introduire les moyens de compression dans le canal et à pousser le bouchon vers l'extrémité distale du canal où il subit une première phase de compression radiale, l'embout amovible est déconnecté de l'appareil, l'embout amovible est connecté à un introducteur comportant des moyens de poussée terminale comprenant au moins deux tiges imbriquées l'une dans l'autre, les moyens de poussée terminale sont actionnés de sorte à déplacer le bouchon vers l'extrémité distale du canal où il subit une deuxième phase de compression radiale.

Les dessins ci-joints sont donnés à titre d'exemples et ne sont pas limitatifs de l'invention. Ils représentent seulement un mode de réalisation de l'invention et permettront de la comprendre aisément.
Figure 1 : Vue en coupe longitudinale du dispositif de délivrance.
   A. Embout amovible non connecté à l'introducteur
   B. Connexion de l'embout à l'introducteur
   C. Ejection du bouchon.
Figure 2 : Vue en coupe longitudinale selon un mode de réalisation de l'appareil destiné à la compression du bouchon et coopérant avec l'embout amovible.
Figure 3 : Vue en coupe longitudinale du dispositif selon un mode de réalisation où les moyens de poussée sont en partie malléables.
Figure 4 : Vue en coupe longitudinale du dispositif selon un mode de réalisation où les moyens de poussée sont solides.
Figure 5 : Vue en coupe longitudinale des moyens d'évacuation du dispositif selon un mode de réalisation où les moyens de poussée sont en partie fluides.

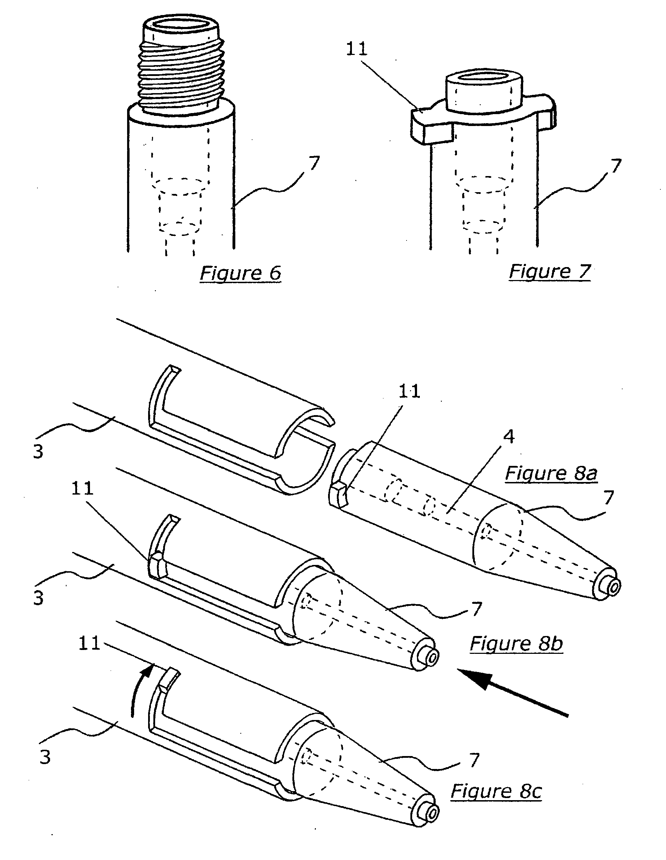

Figure 6 : Vue latérale de l'extrémité proximale de l'embout permettant la connexion par vissage sur l'introducteur.
Figure 7 : vue latérale de l'extrémité proximale de l'embout amovible permettant la fixation sur l'introducteur par baïonnette.
Figure 8 : Cinématique montrant la connexion de l'embout amovible sur l'introducteur au moyen d'une baïonnette.
Figure 9 : Vue en coupe du dispositif selon un mode de réalisation où le canal de guidage est composé d'une succession de compartiments de section décroissante.
Figure 10 : Vue en coupe du dispositif selon un mode de réalisation dans lequel le canal de guidage a une forme de tronc conique.
Figure 11 : Vue en coupe longitudinale du dispositif présentant à son extrémité distale une surface d'appui agrandie.
Figure 12 : Présentation des différentes géométries possibles pour le bouchon d'occlusion.

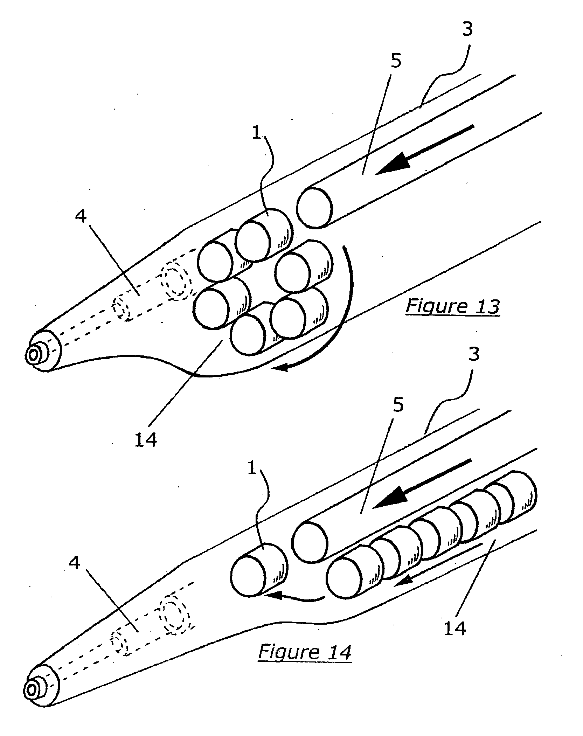

Figure 13 : Vue du dispositif selon un mode de réalisation comprenant un barillet permettant le stockage de plusieurs bouchons.
Figure 14 : Vue du dispositif présentant un logement de stockage permettant de stocker les bouchons les uns derrière les autres.

D'autres buts et avantages apparaîtront au cours de la description qui suit le mode préféré de réalisation de l'invention qui n'en est cependant pas limitatif.

La présente invention concerne un dispositif de délivrance d'un bouchon 1 dans un conduit 2 du corps humain ou animal comprenant un introducteur 3 muni d'un canal 4 de guidage et des moyens pour pousser le bouchon 1 dans le canal 4 caractérisé par le fait que ledit canal 4 est configuré pour comprimer radialement le bouchon 1 au cours de son déplacement vers l'extrémité distale du canal 4.

Avantageusement, le canal 4 est composé d'une succession de compartiments 6.17.18 de section décroissante.

Selon un mode de réalisation, le dernier compartiment est de section légèrement inférieure au diamètre du conduit 2 à occlure. Ainsi le bouchon 1 est mis en place dans le conduit 2 sans endommager les parois.

Avantageusement, le volume de chaque compartiment est constant c'est-à-dire que les sections des compartiments sont décroissantes, la longueur de chaque compartiment est croissante.

Selon un autre mode de réalisation, la réduction de section peut être continue et en particulier réalisée par un profil en forme de tronc de cône.

Avantageusement, le canal 4 se situe à l'extrémité distale dudit introducteur 3 au moins partiellement dans un embout amovible 7.

Ainsi le dispositif de délivrance peut être restérilisable ou à usage unique ou une composition des deux possibilités.

Le canal 4 est composé de trois parties : un compartiment de réception 17 du bouchon 1, un compartiment d'éjection 18 du bouchon 1, et, entre ces deux compartiments, une zone de compression radiale 6. C'est le passage d'un compartiment à l'autre à travers la zone 6 qui comprime radialement le bouchon 1.

Initialement, le bouchon 1 est placé à l'extrémité proximale de l'embout amovible 7 dans le compartiment de réception 17, sous forme sensiblement peu comprimé, voire non comprimé. Son insertion est par conséquent fort pratique.

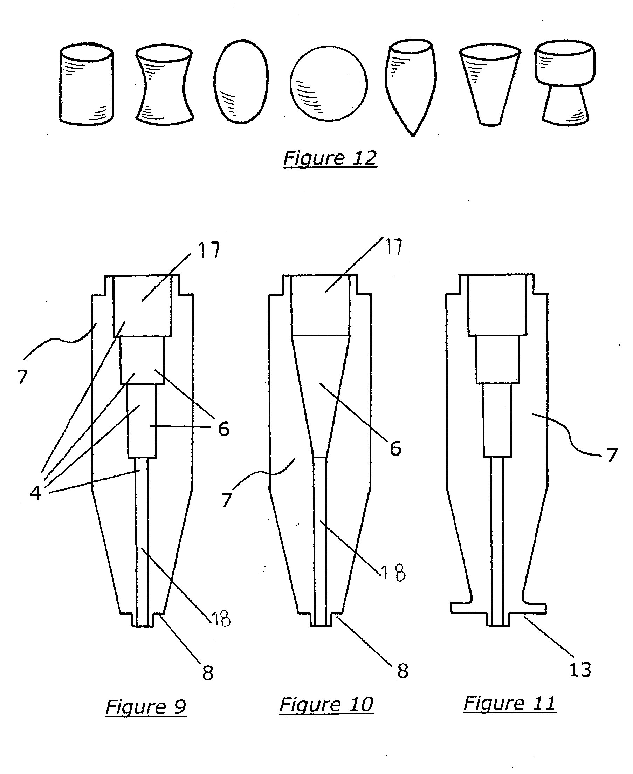

Selon la figure 9, la zone de compression 6 du canal 4 est composée d'une succession de compartiments de section décroissante. Le dernier compartiment, celui d'éjection 18, a la section la plus fine.

Selon la figure 10 la zone de compression 6 du canal 4 est composée d'une portion tronconique.

Selon un mode de réalisation, les moyens pour pousser le bouchon 1 agissent par pression mécanique directe sur le bouchon 1, pour le faire passer du compartiment de réception 17 à celui d'éjection 18, à travers la zone de compression 6. Par pression mécanique directe, on entend que les moyens pour pousser viennent au contact de la surface arrière du bouchon 1.

Selon la configuration du canal 4, les moyens pour pousser ont une section d'appui qui décroît au fur et à mesure de la poussée.

Selon un mode de réalisation privilégié, il s'agit d'une ou plusieurs tiges solides 9 de section décroissante.

De plus, les tiges 9 sont imbriquées pour reproduire une face plane pour pousser le bouchon 1 avec la plus large surface d'appui possible.

Une tige 9 effectue un déplacement longitudinal dans le canal 4 et pousse le bouchon 1 dans le compartiment suivant ou dans la section tronconique. Le bouchon 1 se situe à cette étape comprimé radialement dans le dernier compartiment d'éjection 18.

Avantageusement, l'une des tiges 9 possède une réduction de section à son extrémité distale.

Avantageusement, les moyens pour pousser ont une surface d'appui sur le bouchon 1 proportionnelle à la section du bouchon. Ainsi les moyens pour pousser ne détériorent pas le bouchon 1.

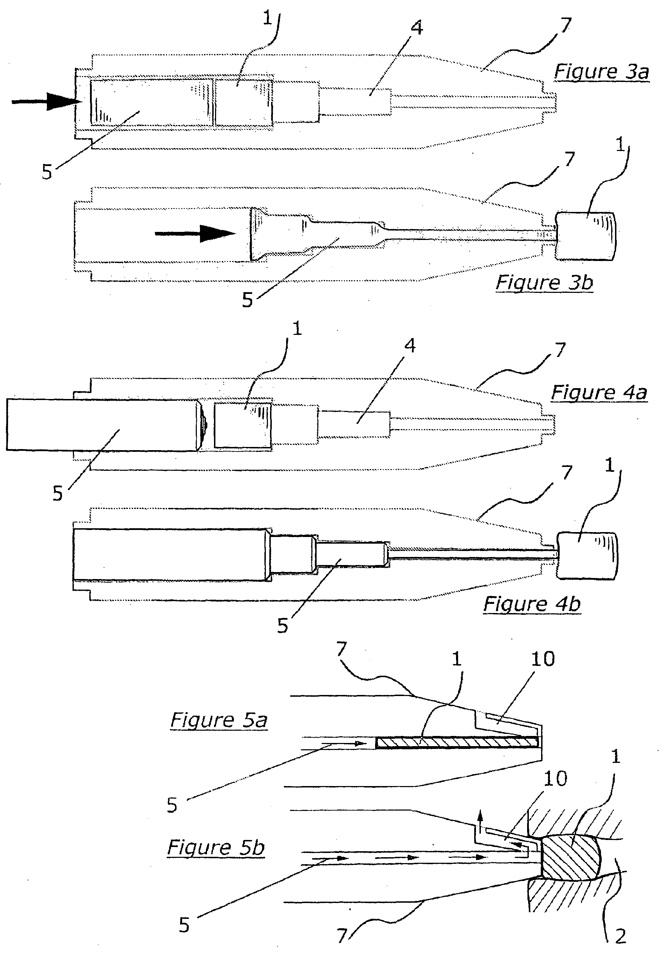

Pour éviter les risques de détérioration du bouchon 1 par des moyens pour pousser solides en contact direct avec le bouchon 1, le dispositif peut être muni de moyens intermédiaires agissant par pression mécanique indirecte. Par pression mécanique indirecte, on entend, par exemple, que les moyens pour pousser sont en partie malléables, comme représenté en figure 3.

Les moyens pour pousser en partie malléables peuvent être à base de silicone ou tout autre matériau déformable.

Ainsi, les moyens pour pousser comprennent un matériau malléable qui transmet l'effort de poussée généré par un fluide tel qu'un gaz ou un liquide ou par un solide tel qu'une tige ou des tiges imbriquées 9.

Selon un autre mode de réalisation, par pression mécanique indirecte, on entend que les moyens pour pousser comprennent un fluide intermédiaire destiné à transmettre l'effort des tiges imbriquées 9 sur ledit bouchon 1.

Le fluide utilisé peut être un liquide ou un gaz. Il peut être compressible ou non.

L'avantage de ce mode de réalisation avec un fluide intermédiaire est de répartir uniformément les forces d'appui des moyens pour pousser sur toute la surface arrière du bouchon 1.

Dans ce cas, les moyens pour pousser doivent être étanches pour éviter tout risque de fuite de fluide et permettre la poussée du bouchon 1.

Ainsi, une tige peut être munie à son extrémité distale de moyens d'étanchéité.

Le bouchon 1 utilisé doit être compatible avec le fluide. C'est-à-dire que le bouchon ne doit pas être dégradé par le fluide et doit être étanche au fluide.

Avantageusement, ledit introducteur 3 est muni à son extrémité distale de moyens d'évacuation 10 destinés à éviter la surpression. Ce moyen d'évacuation 10 est constitué, avantageusement, d'une valve tarée à une pression prédéterminée placée dans un tuyau communiquant à une extrémité avec le canal 4 et à l'autre extrémité avec l'extérieur du dispositif.

Selon un autre mode de réalisation, ce moyen d'évacuation 10 comprend un orifice qui s'ouvre lors du mouvement du piston lors de la compression du bouchon 1.

Le bouchon 1 présente des caractéristiques d'expansion radiale et/ou longitudinale de sorte qu'il soit amené sur le site à occlure dans une position comprimée et que lors de son largage, il reprenne une forme expansée.

Avantageusement, le bouchon 1 est réalisé dans un matériau biocompatible du type polymère tel que le silicone, la mousse de silicone, le polyuréthane, un matériau hydrophile, un polymère à mémoire de forme, hydrogel ou encore biologique tel que le collagène ou encore métallique tel que l'inox ou en alliage en mémoire de forme.

Le bouchon 1 peut avoir différentes géométries de types représentés dans la figure 12 tel qu'un cylindre, un obus, un ovoïde, une sphère, un bouchon de champagne, en T, en cône tronqué, en diabolo, en parapluie.

Ledit bouchon 1 peut présenter des moyens destinés à favoriser son ancrage dans le conduit 2 où il va être implanté (ergots, picots, écailles, stries... )

Selon un mode de réalisation, le bouchon 1 a une extrémité renforcée plus résistante lors de la poussée par les moyens pour pousser 5. L'extrémité renforcée peut être réalisée par traitement thermique ou par un deuxième matériau de caractéristique de dureté plus importante.

La couche renforcée du bouchon 1 doit être suffisante pour résister aux forces des moyens pour pousser. Par exemple, la couche peut représenter 1/10 de l'épaisseur totale du bouchon 1.

La couche renforcée composée d'un matériau plus résistant que le matériau du bouchon 1 peut être rapportée par collage ou par moulage sur ledit bouchon 1.

Selon le mode de réalisation où l'extrémité distale dudit introducteur 3 est un embout amovible 7, avantageusement la connexion entre l'introducteur 3 et son embout amovible 7 est réalisée par une baïonnette. La baïonnette est formée d'une fente à l'extrémité distale dudit introducteur 3 apte à recevoir des ergots de connexion 11 situés sur la partie proximale de l'embout amovible 7 ou inversement.

Selon un autre mode de réalisation, la connexion entre l'introducteur 3 et ledit embout amovible 7 est réalisée par vissage.

D'autres moyens de connexion peuvent être utilisés pour la connexion entre l'introducteur 3 et l'embout amovible 7 tel que clipsage ou tout autre moyen de connexion rapide.

Ledit dispositif de délivrance a pour objectif d'introduire le bouchon 1 dans un conduit 2 du corps humain ou animal. Ainsi, il est fait en matière relativement dure pour supporter l'effort de compression du bouchon 1. Le matériau de l'introducteur 3 peut être du métal ou un polymère, il peut être transparent ou non ou présenter une fenêtre de visualisation pour permettre de voir et contrôler l'éjection du bouchon 1.

Selon un mode de réalisation, l'introducteur 3 est muni d'un ergot destiné à faciliter le positionnement dudit dispositif en regard du conduit 2 à occlure.

Selon un autre mode de réalisation, ledit introducteur 3 est muni à son extrémité distale d'une surface d'appui 8 destinée à permettre un meilleur appui du dispositif sur le pourtour du conduit 2 à occlure.

Avantageusement, cette surface d'appui 8 est une surface d'appui agrandie 13 constituée d'un diamètre plus grand que le diamètre de la fin de l'embout amovible 7 formant ainsi une couronne d'appui.

L'avantage de cette surface d'appui agrandie 13 est de pouvoir contrôler la pénétration du dispositif dans le conduit 2 à occlure.

Dans les cas précédemment décrits, la phase de compression et la phase de poussée finale en vue de la délivrance du bouchon 1 s'opèrent successivement au sein de l'introducteur 3 par une poussée prolongée du bouchon 1 dans le canal 4. Pour une mise en compression radiale par une pression mécanique directe, cela suppose généralement le recours à l'imbrication de plusieurs tiges de poussée. En effet, il faut disposer de tiges de diamètre décroissant pour coopérer avec la plus large surface possible de l'arrière du bouchon 1 tout en passant successivement au travers des compartiments de section décroissante ou de la portion tronconique. Une tige effectue alors le placement en compression du bouchon et une autre tige, apte à une translation longitudinale dans la première, sert à la délivrance du bouchon 1.

Selon un autre mode de réalisation, la phase de compression et la phase de poussée finale ne s'opèrent pas toutes les deux au sein de l'introducteur mais dans deux éléments distincts.

Ainsi, les moyens pour pousser le bouchon 1 comportent dès moyens de compression situés dans un appareil 15 distinct de l'introducteur 3 et des moyens de poussée terminale 5 situés dans l'introducteur 3. L'appareil 15 et les moyens de compression sont aptes à coopérer avec l'embout amovible 7 et sont destinés à comprimer le bouchon 1 dans la partie distale dudit embout amovible 7.

Selon une possibilité, ledit appareil 15 est un support 12 muni d'au moins une tige 16 et avantageusement, deux tiges imbriquées 9 l'une dans l'autre, aptes à coopérer avec ledit embout amovible 7 avant sa connexion avec ledit introducteur 3.

Selon une possibilité, les tiges imbriquées 9 sont situées au niveau de l'appareil 15. Dans ce cas, le bouchon 1 est comprimé dans le canal 4 par l'action des tiges imbriquées 9 dans l'appareil 15 puis l'embout amovible 7 est fixé à l'introducteur 3 qui comprend au moins une tige 9 destinée à éjecter le bouchon 1 en dehors du canal 4.

L'embout amovible 7 est placé tête à l'envers autour des moyens de compression. Il est en appui sur un ressort de rappel qui pousse l'embout amovible 7 en direction opposée au support 12.

Dans le cas où l'embout amovible 7 se connecte à l'introducteur 3 par baïonnette, l'appareil 15 est apte à coopérer avec les ergots de connexion 11 de l'embout amovible 7.

Les ergots ont alors aussi un rôle de maintien dans l'appareil 15.

Lorsque l'embout amovible 7 est placé sur l'appareil 15, il est bloqué en rotation et ne peut en sortir. Le seul moyen de l'en extraire et d'appuyer dessus et de le tourner d'un quart de tour de manière à amener les ergots de l'embout amovible 7 libres de leur butée. Lors de ce mouvement, le bouchon 1 est transféré du compartiment de réception 17 à la zone de compression, voire au compartiment d'éjection 18 par l'action des moyens de compression.

L'avantage de l'appareil 15 est que la compression du bouchon 1 est réalisée seulement quelques secondes avant la mise en place en respectant l'intégrité dudit bouchon 1. Cela permet de conserver au bouchon toutes ses caractéristiques d'élasticité.

Nous allons maintenant décrire plusieurs procédés pour la préparation dudit dispositif de délivrance.

* Selon un mode de réalisation, la compression du bouchon 1 est réalisée par l'appareil 15 muni des moyens de compression avant la connexion de l'embout amovible 7 sur l'introducteur 3.

Le bouchon 1 est tout d'abord inséré dans le compartiment de réception 17, à l'extrémité proximale dudit embout amovible 7, celui-ci est alors placé sur l'appareil 15 muni d'au moins une tige 16. Une pression longitudinale sur ledit embout amovible 7 entraîne l'introduction de la tige 16 des moyens de compression dans le canal 4 de l'embout amovible 7. Le bouchon 1 est alors poussé dans le canal 4 où il subit une compression radiale.

L'embout amovible 7, dont le bouchon 1 est à ce stade comprimé à l'extrémité distale dans le compartiment d'éjection 18, est connecté à l'introducteur 3 par divers moyens de connexion décrits précédemment.

L'actionnement des moyens de poussée terminale 5 va permettre alors l'éjection du bouchon 1 hors du dispositif.

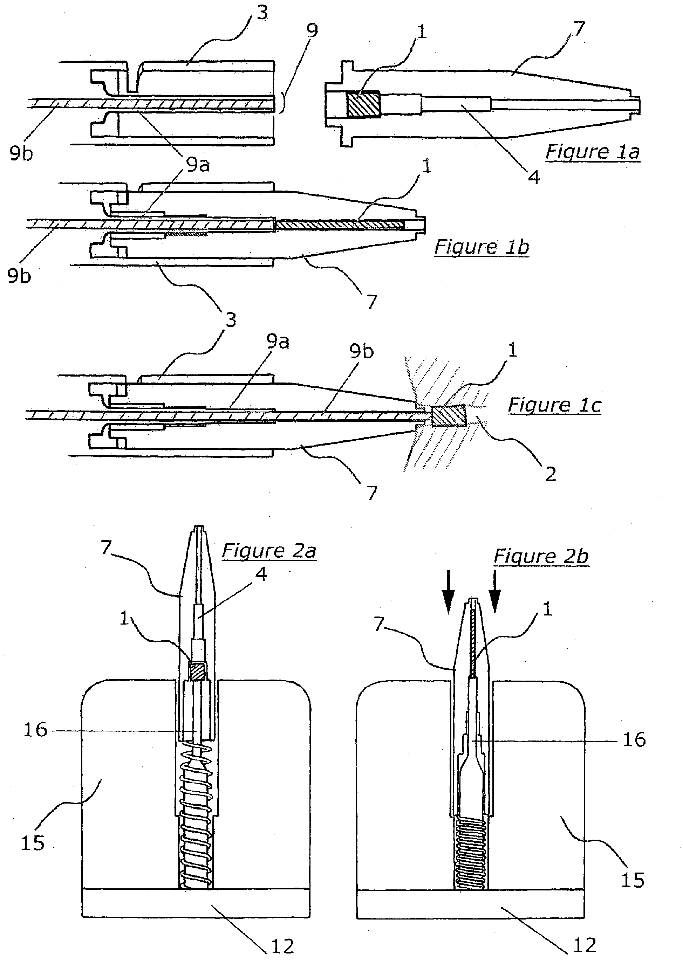

* Selon un autre mode de réalisation privilégié, illustré en figure 1, la compression du bouchon 1 est réalisée lors de la connexion de l'embout amovible 7 sur l'introducteur 3.

Lors de la connexion de l'embout amovible 7, muni du bouchon 1 à son extrémité proximale, sur l'introducteur 3 par divers moyens de connexion décrits précédemment, une tige immobile 9a de l'introducteur 3 appuie sur le bouchon 1 pour le déplacer dans le canal 4 en direction de l'extrémité distale dudit embout amovible 7. Ce déplacement conduit à la compression radiale du bouchon 1.

Puis une tige mobile 9b de l'introducteur 3, imbriqué dans la tige 9a, est actionnée et expulse par poussée le bouchon 1 comprimé hors du dispositif.

* Selon un autre mode de réalisation, la compression du bouchon 1 est réalisée après la connexion de l'embout amovible 7 sur l'introducteur 3. L'embout amovible 7 est connecté à l'introducteur 3 par divers moyens de connexion décrits précédemment.

La compression du bouchon 1 se fait par l'actionnement des moyens pour pousser.

Selon ce mode de réalisation, les moyens pour pousser permettent une compression du bouchon 1 puis son éjection.

Selon ce mode de réalisation, les deux phases de compression et d'éjection peuvent être réalisées par un actionnement unique des moyens pour pousser ou par des actionnements successifs des moyens pour pousser.

Les modes de réalisation précédemment décrits peuvent être utilisés séparément ou en combinaison.

Selon un mode de réalisation, le dispositif de délivrance est un ancillaire ayant la forme classique d'instrument de coelioscopie. Une gâchette donne un mouvement de translation à un tirant compris dans un tube. Lors de l'actionnement de la gâchette le tirant est déplacé (mouvement de translation de la partie proximale du tube vers la partie distale).

Avantageusement, le système peut être lubrifié par un produit visqueux ou par un liquide pour limiter les efforts lors de la compression et de l'éjection.

Selon un mode de réalisation, un logement de stockage 14 peut exister dans le corps de l'introducteur 3. Le logement de stockage 14 permet de stocker plus de un bouchon 1 dans le dispositif sous forme peu ou non comprimé.

Le logement de stockage 14 peut se présenter sous la forme d'un barillet. La rotation du barillet entraîne l'alignement du bouchon 1 par rapport aux moyens pour pousser. La rotation du barillet peut être effectuée manuellement par le praticien ou automatiquement dès que les moyens pour pousser ne sont plus en regard d'un bouchon 1.

Le logement de stockage 14 peut aussi être de forme longitudinale permettant le stockage des bouchons 1 les uns derrière les autres.

Des moyens d'avancement des bouchons peuvent être utilisés pour faire avancer les bouchons 1 et en mettre successivement un en alignement avec les moyens pour pousser.

### REFERENCES

- 1.: Bouchon
- 2.: Conduit du corps
- 3.: Introducteur
- 4.: Canal
- 5.: Moyens de poussée terminale
- 6.: Zone de compression
- 7.: Embout amovible
- 8.: Surface d'appui
- 9.: Tiges 9a : tige immobile
- 9b :: tige mobile
- 10.: Moyens d'évacuation
- 11.: Ergots de connexion
- 12.: Support
- 13.: Surface d'appui agrandie
- 14.: Logement de stockage
- 15.: Appareil
- 16.: Tige de compression
- 17.: Compartiment de réception
- 18.: Compartiment d'éjection

## Revendications

1. Dispositif de délivrance d'un bouchon (1) dans un conduit (2) du corps humain ou animal comprenant un introducteur (3) muni d'un canal (4) de guidage, étant configuré pour comprimer radialement le bouchon (1) au cours de son déplacement vers l'extrémité distale du canal (4), et des moyens pour pousser le bouchon (1) dans ledit canal (4) **caractérisé par le fait**
**que** lesdits moyens pour pousser le bouchon (1) comprennent au moins deux tiges imbriquées (9) aptes à coulisser l'une dans l'autre.

2. Dispositif selon la revendication 1 **caractérisé par le fait**
**que** ledit canal (4) est composé d'une succession de compartiments de section décroissante.

3. Dispositif selon la revendication 1 **caractérisé par le fait**
**que** ledit canal (4) a un profil en forme de tronc de cône destiné à réduire la section dudit canal (4) vers son extrémité distale.

4. Dispositif selon l'une quelconque des revendications précédentes **caractérisé par le fait que**
le canal (4) se situe à l'extrémité distale dudit introducteur (3) au moins pour partie dans un embout amovible (7).

5. Dispositif selon la revendication 4 **caractérisé par le fait**
**que** les moyens pour pousser le bouchon (1) comportent :
- des moyens de compression situés dans un appareil (15) sur lequel l'embout est connectable et,
- des moyens de poussée terminale (5) situés dans l'introducteur (3).

6. Dispositif selon la revendication 4 ou 5 **caractérisé par le fait que** la connexion entre l'introducteur (3) et son embout amovible (7) est réalisée par une baïonnette.

7. Dispositif selon la revendication 4 ou 5 **caractérisé par le fait que** la connexion entre l'introducteur (3) et son embout amovible (7) est réalisée par vissage.

8. Dispositif selon l'une quelconque des revendications précédentes **caractérisé par le fait**
**qu'**une tige (9) possède une réduction de section à son extrémité distale.

9. Dispositif selon l'une quelconque des revendications précédentes **caractérisé par le fait que**
l'introducteur (3) est muni d'un ergot destiné à faciliter le positionnement du dispositif en regard du conduit à occlure.

10. Dispositif selon l'une quelconque des revendications précédentes **caractérisé par le fait que**
l'introducteur (3) est muni à son extrémité distale d'une surface d'appui agrandie (13) destinée à permettre l'appui dudit dispositif sur le pourtour du conduit à occlure (2).

11. Dispositif selon l'une quelconque des revendications précédentes **caractérisé par le fait**
**que** ledit introducteur (3) comprend un logement de stockage (14) destiné à stoker les bouchons (1) avant leur utilisation.

12. Dispositif selon la revendication 11 **caractérisé par le fait**
**que** le logement de stockage (14) comprend un barillet destiné à stocker et à distribuer les bouchons (1).

13. Dispositif selon une quelconque des revendications précédentes **caractérisé par le fait**
**que** les moyens pour pousser le bouchon (1) comprennent un fluide intermédiaire destiné à transmettre l'effort des tiges imbriquées (9) au bouchon (1).

14. Dispositif selon la revendication 13 **caractérisé par le fait**
**que** l'extrémité distale dudit introducteur (3) est munie d'un moyen d'évacuation (10) destiné à éviter les surpressions.

15. Procédé de mise en compression d'un bouchon (1) **caractérisé par le fait qu'**on utilise un dispositif de délivrance selon la revendication 5 et que
- le bouchon (1) est introduit à l'extrémité proximale d'un embout amovible (7) dans un canal (4),
- l'embout amovible (7) est connecté à un appareil (15) comportant des moyens de compression,
- une pression longitudinale est exercée sur l'embout amovible (7) de sorte à introduire les moyens de compression dans le canal (4) et à pousser le bouchon (1) vers l'extrémité distale du canal (4),
- l'embout amovible (7) est déconnecté de l'appareil (15).
- l'embout amovible (7) est connecté à un introducteur (3) comportant des moyens de poussée terminale (5),
- les moyens de poussée terminale(5)sont actionnés de sorte à déplacer le bouchon (1) vers l'extrémité distale du canal (4).

## Claims

1. Device for delivering a plug (1) in a vessel (2) in a human or animal body, including an inserter (3) fitted with a guiding channel (4), being configured to compress radially the plug (1) during the displacement thereof towards the distal end of the channel (4), and means for pushing the plug (1) into the said channel (4) **characterised in that**
said means for pushing the plug (1) consists of two imbricated rods (9), one of which slides in the other.

2. Device according to claim 1 **characterised in that**
said channel (4) consists of a succession of compartments of decreasing section.

3. Device according to claim 1 **characterised in that**
said channel (4) is shaped like a truncated cone and is designed to decrease the section of the said channel (4) towards its distal end.

4. Device according to any of the above claims **characterised in that** the channel (4) is located at the distal end of the said inserter (3) at least partly in a movable tip (7).

5. Device according to claim 4 **characterised in that**
the means for pushing the plug (1) includes the following:
- a compression means located in a device (15) to which the tip can be connected; and
- terminal means for pushing (5) located in the inserter (3).

6. Device according to claim 4 or 5 **characterised in that**
the connection between the inserter (3) and its movable tip (7) consists of a bayonet fitting.

7. Device according to claim 4 or 5 **characterised in that**
the connection between the inserter (3) and its movable tip (7) consists of a screw fitting.

8. Device according to any of the above claims **characterised in that** one rod (9) has a decreased section at its distal end.

9. Device according to any of the above claims **characterised in that**
the inserter (3) is fitted with a spigot to make it easier to position the device in the vessel requiring occlusion.

10. Device according to any of the above claims **characterised in that** the inserter (3) is fitted, at its distal end, with an extended bearing surface (13) to support the said device on the edge of the vessel requiring occlusion (2).

11. Device according to any of the above claims **characterised in that** said inserter (3) includes a storage housing (14) for the storage of plugs (1) before use.

12. Device according to claim 11 **characterised in that**
the storage housing (14) includes a cylinder designed to store and distribute the plugs (1).

13. Device according to any of the above claims **characterised in that** the means for pushing the plug (1) includes an intermediate fluid to transmit stress from the imbricated rods (9) to the plug (1).

14. Device according to claim 13 **characterised in that**
the distal end of the said inserter (3) is fitted with an outlet (10) to avoid excess build-up of pressure.

15. Method for compressing the plug (1) **characterised in that**
an insertion device is used in accordance with claim 5 and that:
- the plug (1) is introduced at the proximal end of a movable tip (7) into a channel (4);
- the movable tip (7) is connected to a device (15) comprising compression means ;
- longitudinal pressure is exercised on the movable tip (7) to introduce the compression means into the channel (4) and push the plug (1) towards the distal end of the channel (4);
- the movable tip (7) is disconnected from the device (15);
- the movable tip (7) is connected to an inserter (3) comprising terminal means for pushing (5);
- the terminal means for pushing (5) are activated in such a way as to displace the plug (1) towards the distal end of channel (4).

## Patentansprüche

1. Ausgabevorrichtung für einen Stopfen (1) in einen Kanal (2) des menschlichen oder tierischen Körpers, bestehend aus einem Einführteil (3) mit einem Führungskanal (4), das derart gestaltet ist, dass der Stopfen (1) bei seiner Bewegung in Richtung distales Ende des Führungskanals (4) radial zusammengepresst wird, **gekennzeichnet dadurch, dass** die Mittel zur Vorwärtsdrücken des Stopfens (1) mindestens zwei sich überlappende Stäbe (9) besitzen, die ineinander gleiten können.

2. Vorrichtung gemäß Patentanspruch 1, **gekennzeichnet dadurch, dass** der Führungskanal (4) aus einer Folge von Abteilen mit abnehmendem Querschnitt besteht.

3. Vorrichtung gemäß Patentanspruch 1, **gekennzeichnet dadurch, dass** der Führungskanal (4) ein kegelstumpfförmiges Profil besitzt, das dazu bestimmt ist, den Querschnitt des Führungskanals (4) in Richtung auf sein distales Ende hin zu verringern.

4. Vorrichtung gemäß einem der vorstehenden Patentansprüche, **gekennzeichnet dadurch, dass** der Führungskanal (4) sich am distalen Ende des Einführteils (3) zumindest teilweise in einem abnehmbaren Endstück (7) befindet.

5. Vorrichtung gemäß Patentanspruch 4, **gekennzeichnet dadurch, dass** die Mittel zum Vorwärtsdrücken des Stopfens (1) folgende Elemente besitzen:
- Mittel zum Zusammenpressen in einem Gerät (15), an welches das Endstück angeschlossen werden kann, und
- Mittel zur abschließenden Druckaufgabe (5), die sich im Einführteil (3) befinden.

6. Vorrichtung gemäß Patentanspruch 4 oder 5, **gekennzeichnet dadurch, dass** die Verbindung zwischen dem Einführteil (3) und seinem abnehmbaren Endstück (7) durch ein Bajonettsystem hergestellt wird.

7. Vorrichtung gemäß Patentanspruch 4 oder 5, **gekennzeichnet dadurch, dass** die Verbindung zwischen dem Einführteil (3) und seinem abnehmbaren Endstück (7) eine Verschraubung ist.

8. Vorrichtung gemäß einem der vorstehenden Patentansprüche, **gekennzeichnet dadurch, dass** ein Stab (9) an seinem distalen Ende eine Querschnittsverringerung aufweist.

9. Vorrichtung gemäß einem der vorstehenden Patentansprüche, **gekennzeichnet dadurch, dass** das Einführteil (3) mit einem Vorsprung versehen ist, um die Positionierung der Vorrichtung vor dem zu verschließenden Kanal zu erleichtern.

10. Vorrichtung gemäß einem der vorstehenden Patentansprüche, **gekennzeichnet dadurch, dass** das Einführteil (3) an seinem distalen Ende mit einer vergrößerten Anlagefläche (13) versehen ist, um das Anlegen der Vorrichtung an dem Umfang des zu verschließenden Kanals zu ermöglichen (2).

11. Vorrichtung gemäß einem der vorstehenden Patentansprüche, **gekennzeichnet dadurch, dass** das Einführteil (3) eine Aufnahmeöffnung (14) besitzt, die zur Lagerung der Stopfen (1) vor ihrer Verwendung bestimmt ist.

12. Vorrichtung gemäß Patentanspruch 11, **gekennzeichnet dadurch, dass** die Aufnahmeöffnung (14) ein Magazin zur Lagerung und Ausgabe der Stopfen (1) besitzt.

13. Vorrichtung gemäß einem der vorstehenden Patentansprüche, **gekennzeichnet dadurch, dass** die Mittel zum Vorwärtsdrücken des Stopfens (1) eine Fluid-Zwischenlage enthalten, um die Kraft der einander überlappenden Stäbe (9) auf den Stopfen (1) zu übertragen.

14. Vorrichtung gemäß Patentanspruch 13, **gekennzeichnet dadurch, dass** das distale Ende des Einführteils (3) mit einer Entleerungseinrichtung (10) versehen ist, die zur Vermeidung von Überdrücken bestimmt ist.

15. Verfahren zum Zusammenpressen eines Stopfens (1), **gekennzeichnet dadurch, dass** eine Ausgabevorrichtung gemäß Patentanspruch 5 verwendet wird und
- der Stopfen (1) am proximalen Ende eines abnehmbaren Endstückes (7) in einen Führungskanal (4) eingeführt wird,
- das abnehmbare Endstück (7) an ein Gerät (15) angeschlossen wird, das Mittel zum Zusammenpressen besitzt,
- auf das abnehmbare Endstück (7) ein Druck in Längsrichtung ausgeübt wird, so dass die Mittel zum Zusammenpressen in den Führungskanal (4) eingeführt werden können und der Stopfen (1) in Richtung distales Ende des Führungskanals (4) vorwärts gedrückt wird,
- das abnehmbare Endstück (7) vom Gerät (15) getrennt ist,
- das abnehmbare Endstück (7) an ein Einführteil (3) angeschlossen wird, das Mittel zur abschließenden Druckaufgabe (5) besitzt,
- die Mittel zur abschließenden Druckaufgabe (5) derart betätigt werden, dass der Stopfen (1) in Richtung distales Ende des Führungskanals (4) bewegt wird.
